Europäisches Patentamt

European Patent Office

Office européen des brevets

(11) Publication number: **0 222 538**
A2

# (12) EUROPEAN PATENT APPLICATION

(21) Application number: **86308234.3**

(22) Date of filing: **23.10.86**

(51) Int. Cl.⁴: **C 10 J 3/46**
**C 10 J 3/54, C 10 J 3/56**

(30) Priority: **04.11.85 GB 8527136**

(43) Date of publication of application:
**20.05.87 Bulletin 87/21**

(84) Designated Contracting States:
**BE DE FR IT NL SE**

(71) Applicant: **British Gas plc**
**Rivermill House 152 Grosvenor Road**
**London SW1V 3JL(GB)**

(72) Inventor: **Timmins, Cyril**
**11 Whitehouse Close**
**Solihull West Midlands(GB)**

(72) Inventor: **Wild, Keith Robert**
**18 Mayama Road Fazeley**
**Tamworth Staffordshire(GB)**

(54) Coal hydrogenation processes.

(57) Solid particulate carbonaceous feed stocks e.g. coal fines and chars are hydrogenated by effecting a first hydrogenation reaction with a hydrogenating gas under entrained flow conditions followed by a second hydrogenation of the solid reactor products and unreacted feedstock in a fluidised bed.

EP 0 222 538 A2

This invention relates to the hydrogenation or hydrogasification 0222538 solid carbonaceous materials. More particularly, the invention relates to the production of methane-containing gases by the thermal hydrogenation of particulate carbonaceous feedstocks such as coal or coal chars. The methane-containing product gas may be used as a fuel gas, as a precursor for a fuel gas of higher calorific value, e.g. substitute natural gas (SNG) or as a synthesis gas used, for example, in the production of hydrogen.

Current research in coal hydrogenation is centered on two types of technology; the entrained flow gasifier, such as developed by the Rockwell Corporation and the fluidised bed reactor, for example, that developed by Rheinische BraunKohlenwerke GmbH. Both reactor types are relatively uncomplicated and each can offer certain advantages. However, there are many disadvantages associated with each reactor type. Thus, with entrained flow reactors, very high preheat and reaction temperatures are required because the plug flow regime limits the residence time of the reactants in the reactor. It is also difficult to effect separation of char from the gas and to subsequently transfer the char, for example to a further gasification stage. There are feedstock restrictions associated with fluidised bed reactors since if the particle size variation is not controlled or the particles have a tendency for caking and agglomeration, the fluidization characteristics of the bed may be adversely affected.

The present invention seeks to provide a process and apparatus therefore in which the disadvantages of the prior are alleviated or even avoided.

Accordingly, the present invention provides a process for the hydrogenation of solid carbonaceous materials wherein a particulate feedstock is reacted with a hydrogenating gas at a temperature of not less than 700°C, characterised in that said particulate feedstock and a first hydrogenating gas are reacted under entrained flow conditions and thereafter at least the solid products and unreacted particulate feedstock are subjected to further hydrogenation in a fluidised bed comprising same with a second hydrogenating gas.

The present invention also provides apparatus for carrying out a two stage hydrogenation reaction including first and second reaction chambers directly communicating with each other, whereas said first chamber includes means for supplying thereto a first hydrogenating gas and means for supplying thereto a suspension of particles of a carbonaceous feedstock in a transport gas and said second chamber includes means for receiving and containing a bed of solid particulate material, means for supplying a fluidising agent and a second hydrogenating gas to the receiving and containing means, and off-take means for removing gaseous products.

The process of the invention may be operated, in the entrained section, by using either plugflow, i.e. single pass, systems or by recirculating systems. Similarly, the arrangement of the fluidized bed section may be such that the bed is stationary, whilst fluidised or, alternatively, the bed may be a recirculating fluidised bed.

The hydrogenating gas may be any gas known per se for hydrogenation, e.g. hydrogen or a hydrogen-containing gas. The same gas may be employed for both the entrained and fluidized zones or different gases may be employed for each zone. In a preferred embodiment the hydrogenating gas is a synthesis gas obtained by the ash-slagging gasification of coal.

The fluidising agent for the fluidised bed section may be an inert gas or may be, at least in part, the hydrogenating gas relevant for the fluidized bed hydrogenation reaction.

The invention will be illustrated in further detail by reference to the accompanying drawings which are a schematic representation of apparatus of the invention entrained and fluidised zones as follows:-

Fig. 1    Recirculating Entrained Flow Zone - Stationary Fluidised Bed Zone.

Fig. 2    Plug Flow Entrained Flow Zone - Stationary Fluidised Bed Zone

Fig. 3    Plug Flow Entrained Flow Zone - Recirculating Fluidised Bed Zone.

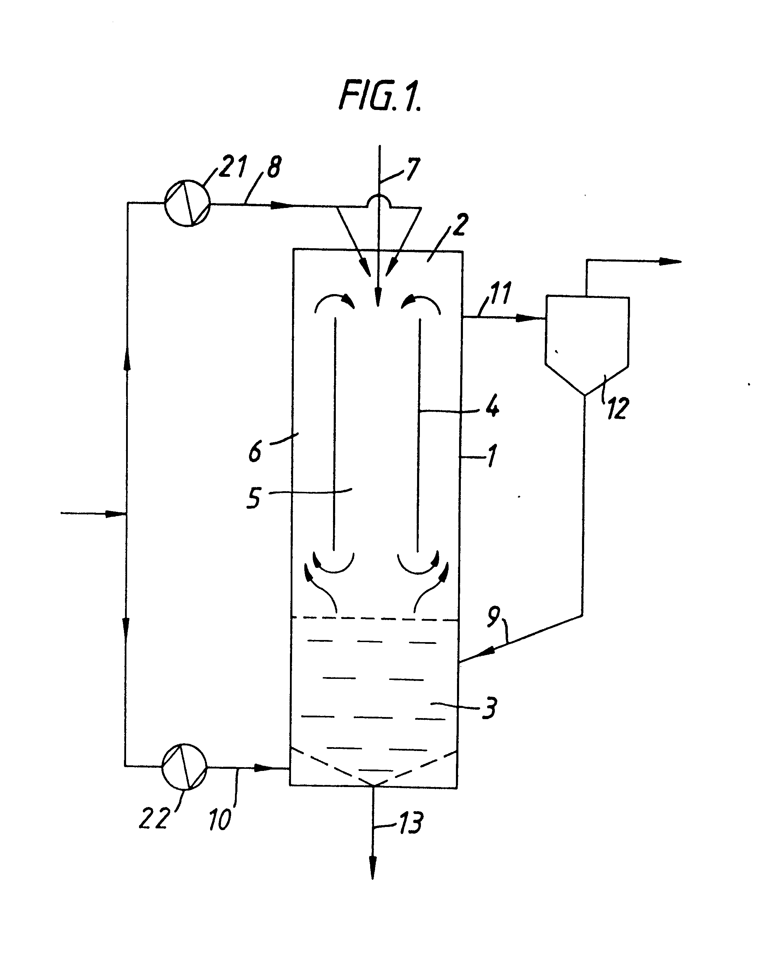

Referring to Figure 1, the hydrogenation or hydrogasifier vessel 1 is provided with two regions, an entrained-flow zone 2, and a fluidised bed zone 3. The entrained flow zone is subdivided into regions by a cylindrical partition 4. The inner region 5 forms a down comer, whereas the outer annular region 6 forms a riser.

Hydrogenating gas for the entrained flow and fluidised bed regions are supplied through lines 8 and 10, respectively. EAch of lines 8 and 10 is provided with a heater, 22 and 22, for independently heating each stream.

Pulverised coal is injected into the down comer via line 7 along with hydrogenating gas through line 8. The arrangement of the inlets for both the coal and the hydrogenating may be such that the hydrogenating gas is employed as the transport phase for the entrained flow regime with the down comer 5. Alternatively, (not shown) the coal particles may be transported in a further supply of hydrogenating gas or inert gas.

Immediately below (downstream) of the entrained flow section is a fluidised bed zone 3, which is fed with char material fed through line 9 and with some material from the downcomer 5.

Fluidisation of zone 3 is maintained by supplying further amounts of hydrogenating gas through line 10 into the base of the bed. In addition to maintaining physical fluidisation of the bed the hydrogenating gas will also react with the carbonaceous bed particles.

Gaseous reaction products and unreacted hydrogenation gas escape from the top of the fluidised bed and rise up the outer annular zone 6. Thus, coal chars, unreacted coal, product gas and unreacted hydrogenating issuing from the bottom of the downcomer tube 4 will mix with off-gases from bed 3 and rise up riser 6.

Gaseous products and some reactants leave the reactor system via gas outlet 11 and cyclone 12. Within the cyclone any solid materials viz.

char or unreacted coal is separated and returned to the fluidised bed zone 3, via line 9. Level control of bed 3 is achieved by removal of bed solids materials through line 13.

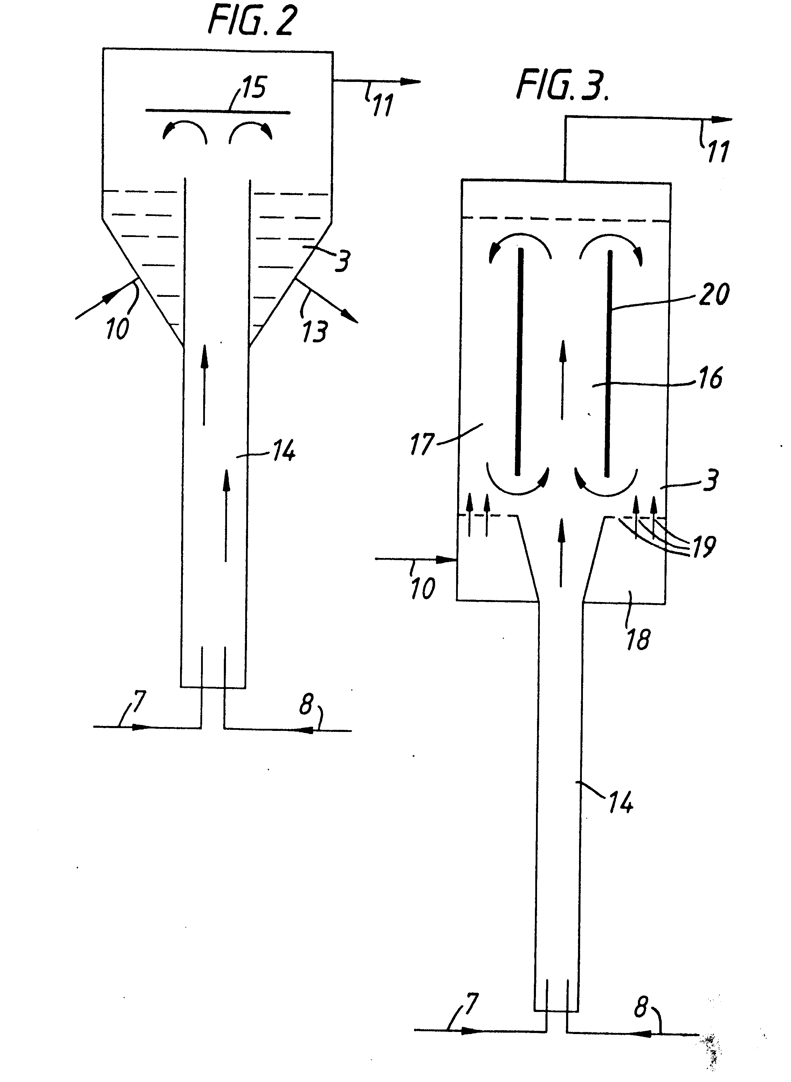

In the embodiment shown in Figure 2 an entrained flow regime is set up in draft-tube 14 which is the mechanical equivalent of the downcomer 5 (Fig. 1); hydrogenating/transport gas and solid reactants being provided through lines 8 and 7, respectively. In this embodiment no provision is made for recirculation within the entrained flow section.

Downstream but above the entrained flow section is provided a fluidized bed section 3. Unreacted coal and coal chars moving up the draft tube 14 encounter baffle 15 and are deflected onto the fluidised bed zone. Fluidising/hydrogenating gas for the bed 3 is provided through line 10 and control of the level of bed 3 is achieved by removal of solids through line 13. Product gas is removed from the reactor systems via line 11. Momentum of inlet coal + $H_2$ causes recirculation.

The arrangement shown in Fig. 3 is, in principle, similar to that of Figure 2 except that the fluidized bed section 3 is a recurculating fluidized bed.

Zone 3 is provided with an internal cylindrical wall 20 which divides the zone into the central cylindrical riser section 16 and an annular downcomer section 17.

Thus reactants and products exiting from the top of the draft tube 14 mix with solid material from the bottom of the downcomer 17 and ascend riser 16. Fluidisation within the riser 16 and velocity of the materials is sustained by the velocity of gas exiting tube 14. Fluidisation and hydrogenation reactions in the annular downcomer 17 is provided by gas supplied through line 10 into chamber 18 and through grid 19; the velocity and pressure of the gas being sufficient to maintain fluidisation but not great enough to affect the downward movement of solid materials in their passage around the bed.

The hydrogenating gas employed in the process may be hydrogen per se or gaseous mixtures comprising hydrogen. An example of such a mixture is the synthesis gas produced by the ash slagging gasification of coal.

Thus, in alternative embodiments of the invention (not shown), rather than supplying hydrogenating gas through line 8 (Figs. 2 & 3), the bottom end of the draft tube is close coupled to the gas outlet of an ash slagging gasifier or other hydrogen generator.

One of the advantages conferred by the process of the present invention is the ability to operate with larger coal particles than is normal for conventional entrained flow reactors such as Rockwell. This is possible because of the fluidised bed stage, which allows longer solids residence times and further gasification. The entrained flow stage operates as a means to preheat the incoming coal so as to provide a hot char supply to the fluidised bed stage and simultaneously rapidly devolatilise and destroy the caking properties of the coal feedstock. For NCB Rossington coal, for example, having about 35% carbon gasification in the entrained flow stage. This devolatilisation should be possible at modest temperatures, typically 750-850°C, even with the larger coal particles. Therefore, for the degree of gasification necessary to leave sufficient ungasified char for hydrogen manufacture, which is about 45%, only a further 15% carbon gasification is required from the fluidised bed stage. Moderate temperatures e.g. 850°C would then be needed without resorting to excessively long solid residence times and consequent large reactors.

The invention will be illustrated by the following Examples.

In each example a reaction was carried out in apparatus as schematically shown in Figure 1. The constant parameters for the hydrogenation reactions are shown in Table 1 and the variable parameters for each Example are shown in Table 2.

TABLE 1

Operating Pressure : 38.5 bar (550 psi)

Reaction Temp.- Fluidised Bed Zone : 850°C

Carbon Gasification - Entrained Flow Zone : 30%

                  - Fluidised Bed Zone : 15%

Hydrogenating Gas - to Entrained Flow Zone: 50%

                - to Fluidised Bed Zone : 50%

Coal Feed Temp. : 150°C

Coal Feed Rate : 45.4 $kghr^{-1}$

Recirculation Ratio(in Entrained Bed Zone): 1:1 (recycle : product)

Hydrogenating Gas Composition (vol.%)

                    $H_2$   : 93.00

                    $CH_4$  : 3.84

                    CO    : 2.36

                    $N_2$   : 0.80

| PARAMETER | EXAMPLE | | | | | |
|---|---|---|---|---|---|---|
| | 1 | 2 | 3 | 4 | 5 | 6 |
| Temp (Heater 21) : °C | 323 | 466 | 640 | 166 | 440 | 741 |
| Temp (Heater 22) : °C | 442 | 423.7 | 404 | 204.3 | 164 | 123 |
| $H_2$/Coal Ratio : | 0.4 | 0.4 | 0.4 | 0.4 | 0.4 | 0.4 |
| Entrained Flow Zone- | | | | | | |
| Inlet Temp :°C | 564 | 622 | 688 | 495 | 571 | 652 |
| Reaction Temp :°C | 750 | 800 | 850 | 750 | 800 | 850 |
| Exit Temp :°C | 773 | 811 | 850 | 771 | 810 | 850 |
| Product Gas Composition | | | | | | |
| $CH_4$ Kg. mil $hr^{-1}$ | 2.02 | 1.99 | 1.96 | 1.69 | 1.66 | 1.61 |
| CO | 0.01 | 0.03 | 0.07 | 0.04 | 0.07 | 0.12 |
| $CO_2$ | Trace | Trace | Trace | Trace | 0.01 | 0.01 |
| $H_2$ | 6.26 | 6.31 | 6.44 | 2.19 | 2.30 | 2.45 |
| $H_2O$ | 0.38 | 0.36 | 0.32 | 0.23 | 0.19 | 0.15 |
| $H_2S$ | 0.01 | 0.01 | 0.01 | 0.01 | 0.01 | 0.01 |
| HCL | Trace | Trace | Trace | Trace | Trace | Trace |
| $N_2$ | 0.09 | 0.09 | 0.09 | 0.06 | 0.06 | 0.06 |

## CLAIMS

1  A process for the hydrogenation of solid carbonaceous materials wherein a particulate feedstock is reacted with a hydrogenating gas at a temperature of not less than 700°C, characterised in that said particulate feedstock and a first hydrogenating gas are reacted under entrained flow conditions and thereafter at least the solid products and unreacted particulate feedstock are subjected to further hydrogeneation in a fluidized bed comprising same with a second hydrogenating gas.

2  A process as claimed in claim 1 wherein said first and second hydrogenating gases have the same composition.

3  A process as claimed in claim 2 wherein said hydrogenating gas is obtained by the ash-slagging gasification of coal in the presence of steam and oxygen.

4  A process as claimed in any one of the preceding claims wherein reactants and products of the first hydrogenation reaction are recycled whilst under entrained flow conditions.

5  A process as claimed in any one of the preceding claims wherein the fluidized bed for the second hydrogenation comprises a recirculatory fluidized bed.

6  A process for the hydrogenation of solid carbonaceous materials according to claim 1 and substantially as hereinbefore described.

7  Apparatus for the hydrogenation of solid carbonaceous materials including first and second reaction chambers directly communicating

including first and second reaction chambers directly communicating with each other, wherein said first chamber includes means for supplying thereto a first hydrogenating gas and means for supplying thereto a suspension of particles of a carbonaceous feedstock in a transport gas and said second chamber includes means for receiving and containing a bed of solid particulate material means for supplying a fluidizing agent and a second hydrogenating gas to the receiving and containing means and off-take for removing gases.

8    Apparatus for the hydrogenation of solid carbonaceous materials according to claim 7 and substantially as hereinbefore described with reference to the accompanying drawings.

FIG.1.

FIG.2

FIG.3.